# EUROPEAN PATENT SPECIFICATION

(11) **EP 2 632 474 B1**
(45) Date of publication and mention of the grant of the patent: **24.08.2016**
(21) Application number: 10787260.8
(22) Date of filing: 26.10.2010
(51) Int. Cl.: A61K 36/28, A61K 36/41, A61K 36/48, A61K 36/87, A61P 3/06

(54) **COMPOSITION FOR FOOD SUPPLEMENT AND ITS USE FOR LOWERING HIGH CHOLESTEROL LEVEL**
ZUSAMMENSETZUNG ZUR NAHRUNGSERGÄNZUNGSMITTEL UND IHRE VERWENDUNG ZUR SENKUNG DER HOHEN CHOLESTERINSPIGEL
COMPOSITION DE SUPPLÉMENT ALIMENTAIRE ET SA UTILISATION POUR BAISSER NIVEAU ÉLEVÉ DE CHOLESTÉROL

(43) Date of publication of application: 04.09.2013
(73) Proprietor: Terezov, Vasil Petrov, 1836 Sofia (BG)
(72) Inventor: Terezov, Vasil Petrov, 1836 Sofia (BG)
(74) Representative: Nesheva, Valentina Velikova
(86) International application number: PCT/BG2010/000020
(87) International publication number: WO 2012/054993

(56) References cited:
- EP-A1- 1 407 679
- RU-C1- 2 112 400
- RU-C1- 2 200 020
- KORMOSH N: "Effect of a combinaiton of extract from several plants on cell-mediated and humoral immunity of patients with advanced ovarial cancer", PHYTOTHERAPY RESEARCH, vol. 20, 2006, pages 424-425, XP002637853,
- MCKAY DIANE L ET AL: "A review of the bioactivity of South African herbal teas: Rooibos (Aspalathus linearis) and honeybush (Cyclopia intermedia)", PHYTOTHERAPY RESEARCH, vol. 21, no. 1, 1 January 2007 (2007-01-01), pages 1-16, XP002500806, JOHN WILEY & SONS LTD. CHICHESTER, GB ISSN: 0951-418X, DOI: 10.1002/PTR.1992
- FENG YONG-HONG ET AL: "Low dose of resveratrol enhanced immune response of mice", ACTA PHARMACOLOGICA SINICA, vol. 23, no. 10, 1 October 2002 (2002-10-01), pages 893-897, XP002378877, NATURE PUBLISHING GROUP, US, CN ISSN: 1671-4083
- PANDEY RUCHI ET AL: "Tribulus terrestris fruit extract protects against oxidative stress-induced apoptosis", BIOSIS,, 1 January 2007 (2007-01-01), XP002607601,
- DATABASE FSTA [Online] INTERNATIONAL FOOD INFORMATION SERVICE (IFIS), FRANkFURT-MAIN, DE; 1985, OBUKHOVA E I ET AL: "Use of tonic plants in the manufacture of new types of aromatized wines. (translated)", XP002637848, Database accession no. FS-1985-10-H-0137 & SADOVODSTVO, VINOGRADARSTVO I VINODELIE MOLDAVII, vol. 39, no. 12, 1984, pages 33-35, TEKHNOLOGO-KONSTRUKTORSKII INST. NP APO 'YALOVENY', USSR

## Description

### FIELD OF THE INVENTION

This invention relates to composition for lowering injurious high levels of total, LDL and HDL cholesterol and triglycerides. People with decreased immune system function, inclined to frequent diseases (flu, colds, etc.) can use it. It is recommended as well before, during, and after antibiotic, chemio-, and X-ray therapies, as well as for all the rest immune decreasing therapies. Elderly people can use it too. It is recommended for preventive prophylaxis in autumn and spring flu seasons, to people with expressed faintness, migraine, chronic fatigue, hepatitis, AIDS, cancer and all other diseases influencing immunity, as well as for general body's strengthening.

### BACKGROUND OF THE INVENTION

Humankind is put to many immune system suppressing factors resulting of nowadays way of life: environment pollution, smoking, hypo and inadequacy alimentation, stress, high levels of background radiation, irregular and incorrect use of antibiotics. The balance of the body functions is seriously disturbed as a whole and on cellular level to produce own antioxidants for maintenance only the optimal and necessary quantity of free radicals. Risk factors cannot be avoided and the question emerges of immune prophylaxis and immune system stimulation when necessary. Natural immune stimulators are clean mountain air, rational feeding, practicing sports, physiotherapy, thalassotherapy, balneological treatment, tisanes, etc. Within nowadays way of life though this is not enough, therefore regular and periodical use of concentrated preparations stimulating and strengthening immune system's functions is necessary. These preparations play the role of external immune stimulators and antioxidants. Means for immune system functions' recovery are used without being coordinated: part of them remove accumulated waste and toxins on cellar level; others work as adaptogens improving body's abilities to respond to stress and correspondingly recover from it. Free radicals number grows with body's general defilement and they attack seriously the immune system. Antioxidants are necessary exactly for internal homeostasis recovery. Regardless of antioxidants number received by the body through food, these substances are insufficient for the neutralization of free radicals that have been formed. Antioxidants overdose though disturbs homeostasis suppressing body's own activity. That is why their quantity in external acceptance has to be optimized. Antioxidants have maximum effect working in pairs or groups. Giving his electron to a free radical the antioxidant oxidizes itself. It is not active any more and needs to be activated to be able to return to its "working" condition. For this reason antioxidants are grouped together to be able to restore one another.

The acceptance of natural vegetable food supplements is recommended for prophylaxis, therapy, and protection from immune system breakdown. Lately, many herbs are applied for immune system stimulation and for easier adaptation to environment conditions because the herbs are relatively harmless, well tolerated, and have low toxicity.

Multiple herbs combinations are known. For example, from BG 804Y a biostimulating food supplement is known based on extracts from *Tribulus terrestris* and roots of *Ginseng,* which acts immune stimulating and strengthening heart-vascular and nervous systems. But it is not intended to free the body from toxins.

It is known from US 2005/0255179 A1 a food supplement for every day use based on rooibos (*Aspalathus* /*inearis*) or honey bush (*Cyclopia intermedia*) or the mixture of both, lycopene, and folic acid (Vitamin B9). This food supplement has a decreased number of ingredients and its main feature is the powerful antioxidant function of rooibos and/or honey bush and lycopene. Folic acid serves to regenerate damaged cells. The preparation though has no ingredient removing waste and toxins from the body and cells.

Another publication WO 0185182 describes combination of cartilage, extract of seeds of grapes, and extract of tomatoes for oral application. This combination though is designed only to increase collagen in skin and decrease free radicals in derma and not for general strengthening of the body.

It is known from MD1378 (F1) about the use of leuzea in medicative food supplement based on a number of herbs to reach immune stimulating and tonic effect. It is a complex mixture of herbs including siberian ginseng and other herbs giving the body a possibility to respond to one or more ingredients. Its effect does not result out of mutual interconnection of all the ingredients to reach common effect of all of them. Here only the roots of leuzea are used and the rest of the plant is practically converted into a technological waste.

It is also known from RU2279837 (C2) a concentrated drink based on vegetable products containing extract of rhizome and roots of leuzea having antioxidant and antistress effect, which main action is directed to systems and glands, but not straight to all the cells in the body. Only rhizome and roots of leuzea are used here too with their antioxidant effect but without using other properties of the herb.

It is known from RU2125815 (C1) complex biologically active food supplement based on vegetal products, containing extract of rhizome and roots of leuzea, extract of rhizome and roots of *Rhodiola* rosea, as well as extracts of other herbs like eleuterococcus, ginseng, schisandra and aralia. In this combination a synergetic effect of herbs extracts is reported. Here as well, only roots of leuzea are used with their antioxidant action without application of properties of the other parts of the herb.

Notwithstanding that some of known herbs combinations potentiate each other's action, the known food supplements have no complex synergetic effect providing simultaneously optimum oxidative stress decrease on cellular level, toxins release and stimulation of the cell activity of immune system.

There are no publications on epigeous parts of leuzea use as a source of fibers and ballast substances for cellular and intercellular depuration of the body. No use of roots of leuzea is known with their antioxidant effect in combination with plant's epigeous parts as fibers and ballast substances source for cellular and intercellular depuration of the body. These effects are potentiateting each other.

It is known from US 2004161524 a method for production of plant extract including the steps of grinding a dry drug, extracting for derivation of active ingredients, and lyophilizing. In this method, a pulverous plant extract is added to a preliminarily prepared plant extract rich on certain active ingredient, and after that the mixture is concentrated and dried. In this known method there are not used completely the active ingredients of the pulverous plant drug. Besides, no preliminary purifying from unwanted pollutants accumulated in the plant is made.

From RU 2174397 a method is known for production of ecdysterone from fresh leaves juice of leuzea, which after filtration and concentration, is purified for about 30 seconds using ethyl acetate. After that the substance undergoes three stages extraction with ethyl acetate organic solvent for Ecdysterone's eduction. The extracts are gathered and heated up for partial removal of ethyl acetate. Obtained concentrate is cooled down to room temperature and filtered to separate ecdysterone crystals. This method purposefully looks for separation of Ecdysterone only. Thus, the other useful ingredients of the plant and the caulis rich on fibers are not utilized.

### SUMMARY OF THE INVENTION

The present invention is defined by the claims. It is an object of the present invention to provide another composition based on plant extracts having complex synergetic effect on lowering injurious high levels of total, LDL, HDL cholesterol and triglycerides; as well as adapted for long-term use without any side effects for the body. Another object of the invention is to create a method for manufacturing composition for food supplement having plant extracts purified from unwanted additions resulting out of environment pollution. These and other problems are solved through a composition for food supplement on plant basis comprising extracts of leuzea (Rhaponticum carthamoides), rhodiola rosea (Rhodiola rosea L), grapes seeds, and green rooibos (Aspalathus linearis). Leuzea extract is derived from a drug obtained from entire plant's parts. The advantage of the proposed food supplement is its increased complex synergetic effect providing simultaneous release of toxins, immune system cellular activity stimulation, optimal oxidizing stress decrease on cellular level, and reduction of total, LDL, HDL cholesterol and triglycerides injurious high levels.

Unexpectedly, it was discovered that in leuzea stem there are many fibers, which stay in the extract after being processed and act on intercellular and cellular level removing waste products and toxins. Removal of poisons and toxins from the body leads to nervous system's recovery, removal of salts, waste, fractions of metabolism, cholesterine; the formation of kidney calculus and gall-bladder is prevented. The composition has relieving effect on entire body and on cardio-vascular system, liver, and pancreas in particular, as well as on the cells themselves. Thus, the environment for free radicals overproduction is cleansed, which are the main source of toxins in the body and the cells; part of them is released at waste removal, they are not piled in the body and their destructive action is eliminated mechanically. At that, adaptogenic and anabolic effect of ecdysterones of leuzea roots is preserved, which is important for immune cells helping and recovery. The extract of rhodiola rosea rich on salidroside is a powerful immune stimulant, appropriate quantities of it help immune system in its strengthening for maintaining body's general homeostasis, as well as the one of different systems and organs, and of the cells in particular. The effect of grapes seeds extract is due mainly to proantocyanide, which is a powerful antioxidant strengthening and improving vessels elasticity and suppressing cholesterol absorption; as well as to resveratrol suppressing tumor growth. Green rooibos extract contains powerful polyphenolic antioxidants including flavonoids and phenolic acids, which in this case are combined and help each other with proantocynide of the grapes seeds. This herbs combination reaches very quick positive response in the body with decreased dose of each of participating ingredients compared to their usual dose. Preferably, the extracts should be dry and proportion in weight % is: leuzea 25-65; grapes seeds 7-30; rhodiola rosea 0.5-2.5; green rooibos 2-10.

In another instance of the invention an extract of tribulus (Tribulus terrestris) is added to the food supplement's composition. It is rich of steroid phyto saponins, mainly protodyoscine and protograciline, as well as the sapogenins diosgenine, tigogenine, hekogenine and chlorogenine. In this case it is put in purposefully as additionally stimulating substance for mental and physical fatigue, to decrease cholesterol levels and regulate hormonal balance. Preferably, the extracts should be dry and their proportion in weight % is: leuzea 25-65; grapes seeds 7-30; rhodiola rosea 0.5-2.5; green rooibos 2-10 and tribulus 7-25.

In the most preferred instance the composition further comprises plant extracts rich of amino acids of proline and lycopene. Unexpectedly, it was discovered that in this combination ingredients strongly potentiate each other at much reduced doses. Within two weeks from the beginning of taking the food suplement a powerful purifying response was observed followed by visual strengthening and improvement of general body's vital functions. This reaction equals to the one to minimum twenty-days-long strict starvation cure avoiding latter's unattractive effects. Preferably, extracts proportion in weight % should be: leuzea 25-65; grapes seeds 7-30; rhodiola rosea 0.5-2.5; green rooibos 2-10; tribulus 7-25 and plant extracts rich of amino acids of proline and lycopene 30-60.

The present disclosure relates as well to a method for manufacturing of food supplement including steps of dry drug grinding, dissolving dry drug, and extraction of unwanted additions and pollutants. Thus, at least part of unwanted accumulations in the plant resulting of environment pollution and human activity are eliminated and the effect of its active substances is improved. Unwanted additions and pollutants are extracted in series of at least two extractions in organic solvent with solvent's concentration 1 :3 and 1 :2 correspondingly, and the drug residue is blew-through to separate the organic solvent. After that the next series is executed with alcohol to remove unwanted additions with solvent's concentration 95%, 70%, and/or 30% correspondingly and the drug residue is dried. Executing extractions on stages leads to maximum purification from unwanted additions and pollutants in plant drug. After it is dried a series of at least three extractions is executed to extract active substances in water; water extracts of all extractions are mixed to obtaining active substances and after that the mixture is filtered. Preferably, after that the mixture is lyophilized. Water extractions made on stages guarantee the drug complete exhaustion and, therefore, its maximum utilization.

Preferably, the drinding of the dry drug is made until particles reach dimensions less than 0.5 mrrr, organic solvent is ethyl acetate and alcohol solvent is ethyl alcohol.

Thus, according to the method purified fractions are obtained with active ingredients' certain contents, which provides for blood's biological purification from oxygen's highly reactive compounds: free radicals and injurious high levels of total, HDL, LDL cholesterol and triglycerides, with strengthening hypotensive action increasing immunity. The advantages of the described method are that it reaches high specificity of the effect; a composition for food supplement is obtained having directed effect; a complete removal of ballast substances is reached; and in the same time the preparation is non-toxic and has no side effects. The composition according to the method is stable in common storage conditions. Its manufacturing is not bounded with thermal processing, which preserves its healing effect. The described method is not labour-intensive and does not require any expensive equipment and chemicals.

The present disclosure relates as well the use of extract of entire plant of leuzea for production of food supplement's composition enriched with ballast substances. This use gives an opportunity to utilize all plant's parts depending on necessities.

### DETAILED DESCRIPTION OF THE INVENTION

In the table below examples are given of compositions according to the invention at 30.00 kg dry drug.

**Table**

| **Ingredients** | **Example 1** | **Example 2** | **Example 3** | **Example 4** | **Example 5** |
|---|---|---|---|---|---|
| Leuzea, weight % | 53,00 | 55,00 | 60,00 | 30,00 | 35,00 |
| Grapes seeds weight % | 20,00 | 20,00 | 25,00 | 7,00 | 10,00 |
| Rhodiola rosea roots weight % | 2,00 | 3,00 | 5,00 | 0,50 | 1,00 |
| Rooibos weight % | 5,00 | 10,00 | 10,00 | 2,00 | 8,00 |
| Tribulus weight % | 20,00 | 12 | - | 7,00 | 10,00 |
| Plant extract containing proline and/or lycopene weight % | - | - | - | 53,50 | 36,00 |

Plant extract containing proline can be an extract of kidney beans or soy beans. Lycopene content can be extracted from tomatoes.

A dry extract has been made according to the invention, as follows: Dry drug, which is a mixture according to examples in the above table was grinded up to dimensions of 0.2-0.5 mm in screw disintegrator. It is double extracted in common conditions (temperature 15-25°C) with ethyl acetate in the ratio of 1:2 and 1:3 in non-corrosive steel extractor to remove substances showing side effects. Organic solvent was eliminated through filtration and organic solvent remains in the drug were eliminated through air blow. The extracted drug was triple processed with 95%, 70% and 30% ethyl alcohol in non-corrosive steel extractor and mixed. After each processing with correspondent aqueous-alcoholic mixture, the obtained alcoholic extract was eliminated. Thus processed drug has been air blown to remove ethyl alcohol. After that the drug was water extracted on stages at least three to five times to its complete exhaustion. Water extracts were mixed, filtered and lyophilized. It was found out that isolated preparations have following physical and chemical characteristics: they are light brown powder with weak to specific plant smell, astringent taste, dissoluble in water completely or partially. As quality ingredients, they are subdivided into: quercetin and kaempferol flavonoid derivatives, polyphenol acids, catechines, antioxidants, fibres, vitamins.

An antioxidants total content analysis of Example 1 has been made by USA BRUNSWICK Laboratories. It has been established that their quantity content is ORAC hydro/µmol TE/g 487.

It has been analyzed samples of Example 4 composition under Book of Hygiene Examinations Methods made by National Foods Hygiene and Medicine Centre in Sofia. It has been established: dry substance 86.6%; general protein 8.8%; citric acid 2.19 mg/g; total fats 0.7%; total ashes 5.8%; fibres 76.6%; general phenols 2564.2 mg of gallic acid /100g; general flavonoids 464.3 mg Catechine equivalent / 100g; vitamin E 3.24 mg/100g; vitamin A 1.75 mg/100g and β carotene 1,31 mg/100g. Energy - 43 kcal/100g.

They have been observed patients who took the composition of Example 4 in 200 g dose for two months twice a day (morning and evening) as a food supplement in gelatinous capsules added to their main treatment. All patients' condition showed general improvement within two weeks.

Other observations have been made too by the Chief of Neurologic Clinic of Military Medical Academy in Sofia over 50 patients suffering of hypercholesterolemia for 12 months. The composition of Example 1 has been given to these patients as a food supplement to the main treatment twice a day - morning and evening, in gelatin capsules in 200 mg doses. Every month, full blood examinations have been done including extended lipidogram and as early as the first month, it has been established a normalization of the high levels of total cholesterol and its fractions as well as triglycerides normalized. The antihypertensive action of the food supplement could not be reported exactly because the patients suffered of arterial hypertension too, which was systematically treated. Control biochemical examinations have been made on the first, sixth and twelfth months of the treatment. All patients' lipidograms stayed in reference values.

They have been observed people freely taking the food supplement (Example 4 composition) in gelatinous capsules in 200 mg dose. Within a month after acceptance, a powerful purifying response starts in kidneys, sinus, constipation retentions, etc.

They have been observed people who in the same conditions used Example 4 and Example 1 compositions one after the other, for one month each. It has been saw durable general condition's stabilization and immunity improvement.

Special observations on blood cholesterol decreasing have been made over patients taking food supplement with Example 4 composition. It has been established a sharp condition's improvement with over 60% decrease of total, LDL and HDL cholesterol and triglycerides within two weeks after acceptance.

**Patient A - Woman (V.T.)**

| **Type of Examination** | **Before acceptance (mmol/L)** | **12 Days after acceptance (mmol/L)** | **Difference (mmol/L)** | **Decrease (%)** |
|---|---|---|---|---|
| **Total Cholesterol** | 6,22 | 4,39 | 1,83 | 70,6 |
| **LDL** | 4,34 | 2,83 | 1,51 | 65,2 |
| **HDL** | 1,45 | 1,25 | 0,20 | 86,4 |
| **Triglycerides** | 0,95 | 0,68 | 0,27 | 71,6 |

**Patient B - Man (G.S.)**

| **Type of Examination** | **Before acceptance (mmol/L)** | **12 Days after acceptance (mmol/L)** | **Difference (mmol/L)** | **Decrease (%)** |
|---|---|---|---|---|
| **Total Cholesterol** | 5,80 | 4,81 | 0,99 | 82,9 |
| **LDL** | 3,73 | 2,83 | 0,90 | 75,9 |
| **HDL** | 1,68 | 1,62 | 0,06 | 96,4 |
| **Triglycerides** | 0,86 | 0,79 | 0,07 | 91,9 |

## Claims

1. Composition for food supplement comprising dry extracts of leuzea (*Rhaponticum carthamoides*) and rhodiola rosea (*Rhodiola rosea L.*) in which at least leuzea extract has been extracted from the entire plant including flower and/or stem with leafs, **characterized by** the fact that it further comprises a dry extract of grapes' seeds and a dry extract of green rooibos (*Aspalathus linearis*) in the following proportion between them in weight %:
- Leuzea 25-65;
- Grapes seeds 7-30;
- Rhodiola rosea 0.5-2.5;
- Green rooibos 2-10.

2. Composition for food supplement according to Claim 1, **characterized by** the fact that it further comprises 7-25 weight % of a dry extract of tribulus *(Tribulus terrestris*).

3. Composition for food supplement according to Claim 2, **characterized by** the fact that it further comprises 30-60 weight % of a dry extract of kidney beans or soy beans and/or extract of tomatoes.

4. Use of composition according to any one of claims 2 or 3 for food supplement for lowering the levels of total, LDL and HDL cholesterol and triglycerides in the blood.

## Patentansprüche

1. Zusammensetzung für eine Nahrungsmittelergänzung umfassend einen Trockenauszug aus Leuzea (*Rhaponticum carthamoides*) und Rhodiolarosea (*Rhodiolarosea L.*), in der mindestens der Trockenauszug aus Leuzea aus der gesamten Pflanze, einschließlich die Blume und/oder der Stamm mit den Blättern, extrachiert ist, **dadurch gekennzeichnet, dass** die Zusammensetzung noch ein Trockenauszug aus Weintraubenkerne und ein Trockenauszug aus grüner Roiboos (*Aspalathus linearis*) in der folgenden Proportion miteinander in Gew. %:
- Leuzea 25-65;
- Weintraubenkerne - 7-30;
- Rhodiolarosea 0.5 - 2.5;
- Grüner Roiboos - 2-10
einschließt.

2. Zusammensetzung für eine Nahrungsmittelergänzung nach Anspruch 1, **dadurch gekennzeichnet, dass** die Zusammensetzung noch 7-25 Gew. % von einem Trockenauszug aus Tribulus *(Tribulus terrestris*) einschließt.

3. Zusammensetzung für eine Nahrungsmittelergänzung nach Anspruch 2, **dadurch gekennzeichnet, dass** die Zusammensetzung noch 30-60 Gew. % von einem Trockenauszug aus Bohnen oder Sojabohnen und/oder Tomaten einschließt.

4. Zusammensetzung für eine Nahrungsmittelergänzung nach einem der Ansprüche 2 oder 3 zur Verwendung bei der Senkung der schädlich hohe Werte von gesamten Cholesterin-, LDL- und HDL- und Triglycerid-Spiegel im Blut.

## Revendications

1. Composition pour un complément alimentaire comprenant des extraits secs de leuzea *(Rhaponticum carthamoides*) et d'orpin rose (*Rhodiola rosea L.)* dans laquelle au moins l'extrait de leuzea a été extraite de la plante entière, y compris la fleur et / ou la tige avec les feuilles, **caractérisée en ce qu'**elle comprend en outre un extrait sec de graines de raisin et un extrait sec de rooibos vert (*Aspalathus linearis*) dans les proportions suivantes en % en poids:
- leuzea 25-65;
- graines de raisin 7-30;
- orpin rose 0,5-2,5;
- rooibos vert 2-10.

2. Composition pour un complément alimentaire selon la revendication 1, **caractérisée en ce qu'**elle comprend en outre de 7 à 25 % en poids d'un extrait sec de tribulus terrestris *(Tribulus terrestris).*

3. Composition pour un complément alimentaire selon la revendication 2, **caractérisée en ce qu'**elle comprend en outre de 30 à 60 % en poids d'un extrait sec d'haricots rouges ou de graines de soja et / ou un extrait de tomates.

4. Composition pour un complément alimentaire selon l'une quelconque des revendications 2 ou 3 pour utilisation dans l'abaissement des niveaux élevés préjudiciables du cholestérol total, de LDL et de HDL et des triglycérides dans le sang.
